Europäisches Patentamt

European Patent Office

Office européen des brevets

(19)

(11) Veröffentlichungsnummer : **0 054 279**
**B1**

(12) **EUROPÄISCHE PATENTSCHRIFT**

(45) Veröffentlichungstag der Patentschrift :
**25.07.84**

(21) Anmeldenummer : **81110373.8**

(22) Anmeldetag : **11.12.81**

(51) Int. Cl.³ : **A 61 K   9/06**, A 61 K   9/70,
A 61 K   9/18

(54) **Präparat mit verzögerter Freisetzung von Nitroglycerin und Verfahren zu dessen Herstellung.**

(30) Priorität : **16.12.80 IL 61721**

(43) Veröffentlichungstag der Anmeldung :
**23.06.82 Patentblatt 82/25**

(45) Bekanntmachung des Hinweises auf die Patenterteilung : **25.07.84 Patentblatt 84/30**

(84) Benannte Vertragsstaaten :
**DE FR GB**

(56) Entgegenhaltungen :
**DE-A- 2 636 559**
**FR-A- 1 589 917**
**FR-A- 2 224 126**
**FR-A- 2 421 610**
**GB-A- 2 021 950**

(73) Patentinhaber : **Forest Laboratorles, Inc.**
**919 Third Avenue**
**New York, N.Y. 10022 (US)**

(72) Erfinder : **Blank, Izhak**
**P.O. Box 664**
**Haifa (IL)**

(74) Vertreter : **Köchling, Conrad, Dipl.-Ing. et al**
**Patentanwälte, Dipl.-Ing. Conrad Köchling Dipl.-Ing.**
**Conrad-Joachim Köchling Fleyer Strasse 135**
**D-5800 Hagen 1 (DE)**

# 0 054 279

**Beschreibung**

Die Erfindung bezieht sich auf Präparate zur topischen Anwendung beim Menschen, die eine vorbestimmte Dosis an Nitroglycerin freisetzen. Die Präparate enthalten stabilisiertes Nitroglycerin in einer Form, die eine langsame und allmähliche Freisetzung von Nitroglycerin ermöglichen, so dass sich die Konzentration des Arzneistoffs im Blut des Patienten über längere Zeit hinweg aufrechterhalten lässt.

Nitroglycerin (Glycerintrinitrat) wird seit vielen Jahren als Koronarvasodilatator verwendet und ist insbesondere geeignet zur Behandlung von Herzerkrankungen, wie Angina pectoris. Nitroglycerin wird im allgemeinen in Form von sublingualen Tabletten oder Kapseln, in Form von Tabletten mit verzögerter Wirkstoffabgabe oder in Form von Salben verwendet.

Nitroglycerin wird durch die Schleimhäute rasch resorbiert. Die Wirkung von sublingualen Tabletten beginnt etwa 3 Minuten nach der Einnahme und dauert etwa 30 Minuten. Die Wirkungsdauer von intern verabreichten Tabletten liegt in der gleichen Grössenordnung.

Die starke Flüchtigkeit und Beweglichkeit von Nitroglycerin führt bei Lagerung und Anwendung zu einer Abgabe des Arzneistoffs an die Umgebung. Werden Tabletten, die Nitroglycerin und einen Trägerstoff, wie Lactose, enthalten, der Atmosphäre in einem halb geschlossenen oder offenen Behälter ausgesetzt, so führt der Verlust an Nitroglycerin zu einer Änderung des Arzneistoffgehalts in den Tabletten. Ferner zeigt sich die Beweglichkeit von Nitroglycerin auch darin, dass es von einer Tablette zur anderen wandern kann, wenn die Tabletten längere Zeit in einem verschlossenen Behälter in Kontakt zueinander kommen.

Die Stabilisierung von Nitroglycerin-Tabletten gegen einen Wirkstoffverlust durch Abgabe an die Atmosphäre und Wanderung von Tablette zu Tablette wurde erreicht, indem man dem zur Herstellung der Tabletten verwendeten Gemisch Polyvinylpyrrolidon einverleibte. Fung et al., Journal of Pharmaceutical Sciences, Bd. 63 (1974), S. 1810 berichten, dass durch Zugabe von Polyvinylpyrrolidon zu Tabletten mit einem Gehalt an Nitroglycerin und Lactose die Geschwindigkeit der Verdampfung von Nitroglycerin aus den Tabletten verlangsamt wird. Jedoch beeinflusst diese Stabilisierung in fester Phase nicht die Verfügbarkeit von Nitroglycerin in einer wässrigen Umgebung, da das Vinylpyrrolidon-Homopolymerisat in Wasser bevorzugt solvatisiert wird und rasch in Lösung geht.

Aus der DE-OS 2 301 664 geht hervor, dass die Lagerstabilität von Nitroglycerin durch Zugabe von Polyvinylpyrrolidon verlängert wird. In der US-PS 4 091 091 ist die Stabilisierung von Nitroglycerin gegen eine Wanderung von Tablette zu Tablette beschrieben, indem man das Gemisch aus wasserlöslichem Trägerstoff und Nitroglycerin mit Polyvinylpyrrolidon versetzt, wobei aber die rasche Löslichkeit der Tablette im Mund beibehalten bleibt.

Nitroglycerin wird auch rasch durch die intakte menschliche Haut resorbiert. Dies ermöglicht eine topische Anwendung in Form von Salben. Nitroglycerin-Salben auf der Basis von Lanolin, Vaseline® oder ähnlichen hydrophoben und praktisch wasserunlöslichen Grundlagen wirken etwa 3 bis 4 Stunden. Werden diese Salben jedoch auf die Haut aufgetragen, geht ein Teil des Nitroglycerins durch Verflüchtigung verloren und die Salbengrundlage wird rasch von der Haut resorbiert.

Aufgabe der Erfindung ist es, stabilisierte Nitroglycerin-Filme oder -Salben zur Verfügung zu stellen, bei denen bei topischer Anwendung eine Verflüchtigung von Nitroglycerin nur in verringertem Masse erfolgt, der Arzneistoff nach Kontakt mit Feuchtigkeit nur langsam freigesetzt wird und sich eine verzögerte Nitroglycerin-Abgabe ergibt, so dass die gewünschte Konzentration des Arzneistoffs im Blut des Patienten über längere Zeit hinweg aufrechterhalten werden kann.

Aus dem Stand der Technik ergibt sich, dass wasserunlösliche Vinylpyrrolidon-Homopolymerisate mit Nitroglycerin in festen Tabletten einen Komplex bilden und die Flüchtigkeit und die Wanderungsfähigkeit des Arzneistoffs verringern. Überraschenderweise wurde nunmehr festgestellt, dass die Einverleibung von wasserunlöslichen Vinylpyrrolidon-Homopolymerisaten oder -Copolymerisaten in Salben oder Filme mit einem Gehalt an Nitroglycerin eine erhöhte Stabilität und eine verringerte Flüchtigkeit von Nitroglycerin bewirken, dagegen aber bei topischer Anwendung auf die Haut eine langsame Freisetzung des Arzneistoffs ermöglichen.

Ferner wurde festgestellt, dass derartige stabile Salben mit verzögerter Wirkstoffabgabe hergestellt werden können, indem man eine Dispersion oder Lösung eines wasserunlöslichen Vinylpyrrolidon-Polymerisats oder -Copolymerisats mit einer Nitroglycerin-Lösung oder -Trituration und einer üblichen hydrophoben Grundlage, wie Vaseline®, oder Lanolin, vermischt.

Es hat sich herausgestellt, dass man durch die erfindungsgemässe Beimischung eines wasserunlöslichen Vinylpyrrolidon-Homopolymerisats oder Copolymerisats zu Arzneipräparaten gelangt, die gegenüber herkömmlichen Präparaten und Salben wesentliche Vorteile mit sich bringen.

Wasserlösliches Polyvinylpyrrolidon ist ein handelsübliches Homopolymerisat, das durch freiradikalische Polymerisation des Monomeren in Wasser oder einem geeigneten Lösungsmittel hergestellt wird. Für die erfindungsgemässen Zwecke geeignetes wasserunlösliches Polyvinylpyrrolidon kann durch Erhitzen von wasserlöslichem Vinylpyrrolidon-Homopolymerisat in Luft auf 150 °C, in Gegenwart von starkem Alkali auf 100 °C oder in Gegenwart einer freiradikalischen Vorläuferverbindung, wie Ammonium-peroxydisulfat, auf erhöhte Temperaturen, beispielsweise auf 90 °C oder mehr, hergestellt werden. Weitere Verfahren zum Unlöslichmachen von wasserlöslichen Homopolymerisaten sind für den Fachmann naheliegend.

2

Ein wasserunlösliches Copolymerisat mit einem Gehalt an mindestens 95 Prozent Vinylpyrrolidon kann entweder direkt durch Polymerisation von Vinylpyrrolidon in Gegenwart eines mehrfach ungesättigten vernetzenden Monomeren, wie einem Dimethacrylat, z. B. Äthylenglykoldimethacrylat, Triäthylenglykoldimethycrylat und Trimethylolpropantrimethycrylat, eine Polyallylverbindung, wie Diäthylenglykolbisallylcarbonat, Triallylglycerin und Triallylcyanurat, oder einem Polymaleinimid, wie Äthylenbismaleinimid, hergestellt werden.

Die erfindungsgemäss verwendbaren wasserunlöslichen Copolymerisate von Vinylpyrrolidon können durch Copolymerisation von Vinylpyrrolidon mit einem oder mehreren geeigneten Comonomeren in Mengenverhältnissen, die zu wasserunlöslichen, unvernetzten Copolymerisaten führen, hergestellt werden. Beispiele für geeignete Comonomere sind Acrylester, Methacrylester, Vinylester, Crotonsäureester, Vinyläther, Maleinsäurehalbester und -diester, Vinylencarbonat, Styrol, Allylester und Allyläther. Andere Comonomere, die zur Copolymerisation mit Vinylpyrrolidon geeignet sind und dem Fachmann geläufig sind, können ebenfalls verwendet werden. Aus toxikologischen Gesichtspunkten ist die Wahl der Monomeren auf diejenigen beschränkt, die zu Copolymerisaten führen, die bei topischer Anwendung auf die Haut keine toxikologischen Nebenwirkungen hervorrufen.

Die Acrylsäure-, Methacrylsäure-, Crotonsäure- und Maleinsäureester, die zur Herstellung der erfindungsgemäss geeigneten wasserunlöslichen Vinylpyrrolidon-Copolymerisate verwendet werden können, umfassen die Ester von $C_1$-$C_{40}$ linearen, verzweigten oder cyclischen Alkanolen, Aralkanolen, Phenolen und substituierten Phenolen. Die Copolymerisate von Vinylpyrrolidon und Acrylsäure-, Methacrylsäure-, Crotonsäure- und Malein säureestern können durch Copolymerisation von Vinylpyrrolidon mit den entsprechenden Estern oder durch Veresterung der Copolymerisate von Vinylpyrrolidon und Acrylsäure, Methacrylsäure, Crotonsäure und Maleinsäure oder deren Anhydriden mit der entsprechenden Hydroxylverbindung hergestellt werden.

Beispiele für Vinylester und Allylester, die zur Herstellung der erfindungsgemäss geeigneten wasserunlöslichen Vinylpyrrolidon-Copolymerisate verwendet werden können sind Ester von $C_1$-$C_{40}$ linearen, verzweigten oder cyclischen aliphatischen, araliphatischen oder aromatischen Carbonsäuren. Die Copolymerisate von Vinylpyrrolidon und Vinylestern können durch Copolymerisation von Vinylpyrrolidon mit dem entsprechenden Vinylester, durch Umesterung von Copolymerisaten von Vinylpyrrolidon und Vinylacetat oder anderen Vinylestern oder durch Veresterung von hydrolysierten Copolymerisaten von Vinylpyrrolidon und Vinylacetat oder andern Vinylestern hergestellt werden. Copolymerisate von Vinylpyrrolidon und Allylestern können entweder durch direkte Copolymerisation oder durch Umesterung oder Veresterung analog der Herstellung von Vinylester-Copolymerisaten mit Vinylpyrrolidon hergestellt werden.

Pfropfcopolymerisate, die durch Pfropfen von Vinylmonomeren auf Polyvinylpyrrolidon erhältlich sind, können ebenfalls verwendet werden, beispielsweise Pfropfcopolymerisate von Polyvinylpyrrolidon mit Acrylsäureestern, Methacrylsäureestern, Styrol und Vinylacetat.

Ein vernetzendes Monomeres der vorstehend beschriebenen Art kann zur Herstellung der erfindungsgemäss geeigneten Copolymerisate und Pfropfcopolymerisate von Vinylpyrrolidon verwendet werden. Weitere Comonomere, wie ungesättigte Carbonsäuren, beispielsweise Acrylsäure, Methacrylsäure, Crotonsäure und Maleinsäure sowie deren Anhydride, können den Copolymerisaten einverleibt werden. Die unvernetzten Copolymerisate und Pfropfcopolymerisate von Vinylpyrrolidon können in Gegenwart von Peroxiden oder unter Bestrahlung nach üblichen Verfahren vernetzt werden.

Die erfindungsgemäss geeigneten wasserunlöslichen Copolymerisate von Vinylpyrrolidon lassen sich nach üblichen Verfahrensweisen herstellen, beispielsweise durch Polymerisation in Masse, Lösung, Emulsion, Suspension oder Dispersion. Hierzu können geeignete freiradikalische Katalysatoren, wie Peroxyverbindungen, Azoverbindungen, Redoxsysteme, Bestrahlung und andere katalytische Techniken zur Einleitung einer freiradikalischen Polymerisation, eingesetzt werden. Da das Polymerisationsverfahren an sich nicht erfindungswesentlich ist, können beliebige, dem Fachmann geläufige Verfahren angewandt werden.

Die Menge der Comonomeren in den wasserunlöslichen Vinylpyrrolidon-Copolymerisaten kann von 0,1 bis 90 Gewichtsprozent variieren. Die tatsächliche Menge wird aufgrund der Natur des Comonomeren und der Konzentration, die zur Bildung eines wasserunlöslichen Copolymerisats erforderlich ist, entweder vor oder nach der Copolymerisation bestimmt.

Die stabilisierten Nitroglycerin-Präparate, die erfindungsgemäss geeignet sind und eine verzögerte Wirkstoffabgabe gewährleisten, lassen sich herstellen, indem man das wasserunlösliche Vinylpyrrolidon-Homopolymerisat oder -copolymerisat in einem Lösungsmittel, wie Isopropanol, Äthanol oder einem Alkohol-Wasser-Gemisch, löst oder dispergiert und die Polymerlösung oder -dispersion mit einer Nitroglycerinlösung in Äthanol oder einer Nitroglycerintrituration aus Nitroglycerin und einem oder mehreren Trägerstoffen aus der Gruppe Lactose, β-Lactose, Milchzucker, Fructose, Maltose, Saccharose, Mannit oder Sorbit vermischt.

Die Lösung mit einem Gehalt an unvernetztem wasserunlöslichem Vinylpyrrolidon-Copolymerisat und Nitroglycerin kann in Abwesenheit oder Gegenwart eines Trägerstoffs auf eine geeignete Oberfläche gegossen werden, wonach das Lösungsmittel unter Umgebungsdruck oder unter vermindertem Druck bei Umgebungstemperatur oder unter leicht erhöhten Temperaturen abgedampft wird. Der Film auf der Substratoberfläche oder der nach dem Entfernen von Substrat erhaltene Film enthält stabilisiertes Nitroglycerin und kann zu Streifen zerschnitten werden, die zur Erzielung einer verzögerten Wirkstoffabgabe auf die Haut von Patienten aufgelegt werden können.

Die Lösung mit einem Gehalt an unvernetztem, wasserunlöslichen Vinylpyrrolidon-Copolymerisat und Nitroglycerin kann direkt auf die Haut aufgebracht werden, wonach nach Abdampfen des Lösungsmittels ein Film mit einem Gehalt an stabilisiertem Nitroglycerin auf der Haut entsteht. Der Wirkstoff wird langsam aus dem Film freigesetzt und von der Haut des Patienten resorbiert.

Ferner kann eine Lösung oder Dispersion mit einem Gehalt an unvernetztem, wasserunlöslichem Vinylpyrrolidon-Copolymerisat und Nitroglycerin oder eine Dispersion mit einem Gehalt an vernetztem Vinylpyrrolidon-Homopolymerisat oder -Copolymerisat und Nitroglycerin in Abwesenheit oder Gegenwart eines Trägerstoffs auf ein poröses, mit Öffnungen versehenes Substrat, wie Gaze, Verbandstoff oder Verbandpapier, aufgebracht werden, wobei man nach dem Abdampfen des Lösungsmittels ein imprägniertes Produkt mit einem Gehalt an stabilisiertem Nitroglycerin erhält. Nach topischer Anwendung auf die Haut von Patienten wird das Nitroglycerin innerhalb eines verlängerten Zeitraums aus diesem Produkt freigesetzt.

Die Lösung oder Dispersion mit einem Gehalt an wasserunlöslichem Vinylpyrrolidon-Homopolymerisat oder -Copolymerisat und Nitroglycerin kann auch unter Rühren mit einer oder mehreren Salbengrundlagen, wie Petrolatum, Vaseline, Lanolin, Stearin, Walrat oder anderen wachs- oder fettartigen Materialien vermischt werden. Die Salbe kann direkt auf die Haut aufgetragen werden. Eine andere Möglichkeit besteht darin, einen Träger, beispielsweise eine Bandage oder ein Band aus polymerem Material zu beschichten und für die topische Anwendung auf der Haut des Patienten einzusetzen. Das stabilisierte Nitroglycerin wird langsam innerhalb von 24 Stunden freigesetzt und von der Haut des Patienten resorbiert.

Das Nitroglycerin in den erfindungsgemässen Präparaten aus Vinylpyrrolidon-Homopolymerisat oder -Copolymerisat und Nitroglycerin wird langsam freigesetzt und direkt von der Haut des Patienten resorbiert. Obgleich das Vinylpyrrolidon-Homopolymerisat oder -Copolymerisat wasserunlöslich ist, führt die hydrophile Beschaffenheit des darin enthaltenen Vinylpyrrolidons zu einer Absorption von Feuchtigkeit, beispielsweise vom Schweiss auf der Haut des Patienten, und zu einer Extraktion des Nitroglycerins aus dem Präparat, wonach sich die Resorption des Arzneistoffes durch die Haut anschließt. Die Extraktionsgeschwindigkeit kann innerhalb eines breiten Bereiches variiert werden und hängt von der Konzentration des Vinylpyrrolidons im Copolymerisat und/oder vom Grad der gegebenenfalls vorliegenden Vernetzung im Homopolymerisat oder Copolymerisat ab.

Die Konzentrationen an wasserunlöslichem Vinylpyrrolidon-Homopolymerisat oder -Copolymerisat und an Nitroglycerin in den stabilisierten Präparaten der Erfindung können je nach der gewünschten Freisetzungsgeschwindigkeit stark variieren. Die Konzentration an Nitroglycerin kann von etwa 1 bis 6 Prozent, bezogen auf das Gesamtgewicht des Präparats, varriieren, während die Konzentration an wasserunlöslichem Vinylpyrrolidon-Homopolymerisat oder -Copolymerisat von etwa 5 bis etwa 500 Prozent, bezogen auf das Gewicht des Nitroglycerins, schwanken kann.

Die Beispiele erläutern die Erfindung.

Beispiel 1

Ein unvernetztes Vinylpyrrolidon (VP)-Homopolymerisat (Zur Darstellung des Standes der Technik), ein vernetztes Vinylpyrrolidon-Äthylenglykoldimethacrylat (VP/EGDM)-Copolymerisat (Gewichtsverhältnis 98 : 2), ein unvernetztes Vinylpyrrolidon-Laurylmethacrylat (VP/LM)-Copolymerisat (Gewichtsverhältnis 70 : 30) und ein unvernetztes Vinylpyrrolidon-Vinylacetat (VP/VA)-Copolymerisat (Gewichtsverhältnis 79 : 21) werden jeweils durch Lösungspolymerisation in Cyclohexan unter Verwendung von Lauroylperoxid als Katalysator hergestellt. Das viskose Reaktionsgemisch wird auf eine Polytetrafluoräthylenfolie gegossen. Der nach dem Abdampfen des Lösungsmittels erhaltene dünne Film wird bei 60 °C in einem Trockenschrank mit Gebläse getrocknet, von gegebenenfalls vorhandenem restlichem Monomerem durch Waschen mit Petroläther befreit und unter vermindertem Druck bei 60 °C wieder getrocknet.

Zur Herstellung von Salben werden die verschiedenen Polymerisate in Äthanol gelöst oder suspendiert. Eine Äthanollösung mit einem Gehalt an 10 Prozent (Gewicht/Volumen) Nitroglycerin (NG) oder ein Nitroglycerin-Lactose-Pulver mit einem Gehalt an 10 Gewichtsprozent Nitroglycerin werden langsam unter Rühren zur Polymerisatlösung oder -suspension gegeben. Sodann wird das homogene Polymerisat-Nitroglycerin-Gemisch unter Rühren mit Vaseline versetzt. Die fertige Salbe enthält 20 Prozent Polymerisat und 4 Prozent Nitroglycerin. Zu Vergleichszwecken wird eine Salbe ohne Polymerisat mit einem Gehalt an 4 Prozent Nitroglycerin hergestellt.

Um die Stabilität des Nitroglycerins gegen Verdampfung unter drastischen Bedingungen zu ermitteln, werden Salbenproben auf Filterpapierstücke der Abmessungen 10 × 10 cm aufgetragen. Die beschichteten Papierstücke werden mehrere Tage aufgehängt, bis das Äthanol abgedampft ist. Proben mit einem Gehalt an 4 Prozent Nitroglycerin werden bei 45 °C in einem Trockenschrank mit Gebläse aufbewahrt. Die Proben werden nach 4 1/2 bis 17 Tagen entfernt und auf ihren Nitroglyceringehalt untersucht.

Um die Geschwindigkeit der Abgabe von Nitroglycerin an ein wässriges Medium zu bestimmen, werden Proben des beschichteten Filterpapiers, auf die getrocknete Salben mit einem Nitroglyceringehalt von 4 Prozent aufgebracht sind, zusammengefaltet und in 250 ml fassende Erlenmeyer-Kolben mit einem Gehalt an 100 ml entionisiertem Wasser gegeben. Die Kolben werden in ein auf 36 °C thermostatisiertes Schüttelbad gestellt. Proben der flüssigen Phase werden nach Schüttelzeiten von 15, 30 und/oder 60 Minuten zur Durchführung einer Nitroglycerinanalyse entnommen.

Die Nitroglycerinanalysen werden spektrophotometrisch gemäss Fung et al., J. Pharm. Sci., Bd. 63 (1974), S. 1810, (modifiziert) durchgeführt.

Die Werte für die Stabilität des Nitroglycerins gegen Verdampfung bei einer thermischen Alterung bei 45 °C und die Geschwindigkeit der Abgabe von Nitroglycerin an Wasser bei 36 °C sind in Tabelle I zusammengestellt.

Tabelle I

| Polymerisat | keines | VP | VP/EGDM 98/2 | VP/LM 70/30 | VP/LM 70/30 | VP/VA 79/21 |
|---|---|---|---|---|---|---|
| Salbe | | | | | | |
| Polymerisat, % | 0 | 20 | 20 | 20 | 20 | 20 |
| NG, % | 4 | 4 | 4 | 4 | 4* | 4 |
| thermische Alterung bei 45°C restliches NG, % bezogen auf den Anfangsgehalt | | | | | | |
| nach 0 Tagen | 100 | 100 | 100 | 100 | 100 | 100 |
| 4,5 " | 0 | 100 | | | | 100 |
| 7 " | | | 77 | 42 | 49 | |
| 8 " | | | | | | 83 |
| 17 " | | 93 | 76 | 13 | 20 | 77 |
| Extraktion mit Wasser bei 36°C extrahiertes NG, % bezogen auf den Anfangsgehalt | | | | | | |
| nach 15 Minuten | | 48 | 82 | 45 | 0 | 0 | 58 |
| 30 " | | 57 | 100 | | 28 | 7 | |
| 60 " | | | | 65 | 31 | 29 | 71 |

* NG zugesetzt als 10 % NG in Lactose.

Die Ergebnisse in Tabelle I zeigen, dass die Kontrollsalbe ohne Polymerisat nach 4 1/2-tägiger Behandlung im Trockenschrank mit Gebläse bei 45 °C das gesamte Nitroglycerin verloren hat, während in Wasser von 36 °C nach 15 bzw. 30 Minuten 48 bzw. 57 Prozent des Nitroglycerins freigesetzt sind. Die Salbe mit einem Gehalt an wasserlöslichem unvernetztem Polyvinylpyrrolidon weist nach 17-tägiger Behandlung im Trockenschrank noch 93 Prozent des ursprünglichen Nitroglyceringehalts auf, während das gesamte Nitroglycerin nach 30-minütiger Behandlung in Wasser freigesetzt ist. Die Salbe mit einem Gehalt an wasserunlöslichem vernetztem VP/EGDM-Copolymerisat (98 : 2) weist nach 17-tägiger Behandlung im Trockenschrank noch 76 Prozent des Nitroglyceringehalts auf und setzt bei 15-bzw. 60-minütiger Behandlung in Wasser 45 bzw. 65 Prozent des Nitroglycerins frei. Die Salbe mit einem Gehalt an wasserunlöslichem, unvernetztem VP/LM-Copolymerisat (70 : 30) ist gegen eine thermische Alterung weniger stabil als die Salbe mit vernetztem VP/EGDM, setzt aber etwa nach 60-minütiger Behandlung in Wasser nur etwa 30 Prozent des Nitroglycerins frei. Die Salbe mit einem Gehalt an wasserunlöslichem VP/VA-Copolymerisat (79 : 21) verhält sich ähnlich wie die Salbe mit einem Gehalt an vernetztem Polyvinylpyrrolidon. 77 Prozent des Nitroglycerins sind nach 17-tägiger Behandlung im Trockenschrank noch vorhanden und 58 bzw. 71 Prozent des Nitroglycerins werden nach 15- bzw. 60-minütiger Behandlung in Wasser freigesetzt.

# 0 054 279

## Beispiel 2

Ein Vinylpyrrolidon/2-äthylhexylacrylat-Copolymerisat (Gewichtsverhältnis 64:36) wird in Isopropanollösung unter Verwendung von Lauroylperoxid als Katalysator hergestellt. 4,5 g einer 60-prozentigen Lösung des obigen VP/EHA-Copolymerisats in Isopropanol werden mit 12 ml einer 10-prozentigen (Gewicht/Volumen) Lösung von Glycerintrinitrat in Äthanol vermischt. Nach Zumischen von 42 g weissem Petrolatum erhält man eine Salbe mit einem Nitroglyceringehalt von 2 Prozent.

9 Patienten, die an Erkrankungen der Koronararterien und Angina pectoris leiden, werden einer klinischen Untersuchung unterzogen. Aus einer Metalltube wird die Salbe in einem 5 bis 10 cm langen Stück auf den Unterarm der Patienten aufgebracht. Anschliessend wird die Stelle mit einer undurchlässigen Kompresse abgedeckt und die Kompresse befestigt. Die Wirkung der Salbe wird durch Radioisotopentechnik getestet, wobei Multigate-Gleichgewichts-Blutvolumina (multigated equilibrium blood volumes) verwendet werden. Die ersten 5 Patienten werden 5 Stunden nach dem Auftragen der Salbe untersucht. Es ergibt sich eine signifikante Besserung des Ausstosses der linken Herzkammer. Das diastolische Volumen im Ruhezustand und das systolische Volumen im Ruhezustand und bei Belastung nehmen signifikant ab. Sämtliche 9 Patienten werden 24 Stunden nach dem Auftragen der Salbe untersucht. Es ergeben sich sowohl im Ruhezustand als auch bei Belastung verbesserte Ausstossfraktionen und verringerte diastolische und systolische Volumina sowohl im Ruhezustand als auch bei Belastung. Somit wird durch Auftragen der Salbe mit einem Gehalt an 2 Prozent Nitroglycerin und dem VP/EHA-Copolymerisat bereits 5 Stunden nach der Anwendung eine günstige Wirkung auf das kardiovaskuläre System hervorgerufen, die mindestens 24 Stunden anhält.

## Beispiel 3

Ein Vinylpyrrolidon-Laurylmethacrylat-Acrylsäure-Copolymerisat (Gewichtsverhältnis 62,5 : 32,5 : 5) wird durch Polymerisation in Cyclohexan unter Verwendung von Lauroylperoxid als Katalysator hergestellt. Das Polymerisat wird gemäss Beispiel 1 isoliert. Sodann wird das Polymerisat in Äthanol gelöst und mit einer 10-prozentigen (Gewicht/Volumen) Lösung von Nitroglycerin in Äthanol vermischt. Man erhält eine Lösung mit einem Gehalt an 15 Prozent VP/LM/AA-Copolymerisat und 3 Prozent Nitroglycerin. Die Lösung wird auf eine Baumwollbinde gegossen und an der Luft getrocknet. Die Binde wird auf den Unterarm eines Patienten mit Angina pectoris aufgebracht. Die erzielte Beseitigung der Symptome hält mehr als 24 Stunden an.

## Beispiel 4

Ein Vinylpyrrolidon-Laurylmethacrylat-Acrylsäure-Copolymerisat (Gewichtsverhältnis 42,5:52,5:5) wird gemäss Beispiel 3 hergestellt. Eine Äthanollösung mit einem Gehalt an 10 Prozent VP/LM/AA-Copolymerisat und 3 Prozent Nitroglycerin wird mit Lanolin vermischt. Die erhaltene Salbe wird zur Imprägnierung von Gaze verwendet. Nach dem Trocknen wird die imprägnierte Gaze mit einem silikonbeschiehteten Abziehpapier bedeckt und in einen mehrlagigen, heissgesiegelten Beutel, der eine Schicht aus Aluminiumfolie aufweist, gepackt. Bei Bedarf wird die imprägnierte Gaze aus dem Vorratsbeutel entnommen und auf den Arm von Patienten mit Symptomen von Angina pectoris aufgebracht. Die Symptome verschwinden in etwa 2 Stunden. Die Besserung hält 24 Stunden an.

## Beispiel 5

Ein Vinylpyrrolidon-Laurylmethacrylat-Copolymerisat (Gewichtsverhältnis 90 : 10) sowie ein entsprechendes Copolymerisat mit einem Gewichtsverhältnis von 10 : 90 werden durch Lösungspolymerisation in Cyclohexan unter Verwendung von Lauroylperoxid als Katalysator hergestellt.

Bei dem isolierten 90 : 10-VP/LM-Copolymerisat handelt es sich um einen spröden Feststoff, der mit Nitroglycerin-Lactose-Pulver in äthanolischer Lösung vermischt wird. Die erhaltene Lösung mit einem gehalt an 3 Prozent nitroglycerin wird zur Imprägnierung einer Gazebinde verwendet. Die getrocknete, imprägnierte Binde bewirkt eine Beseitigung der Symptome von Angina pectoris innerhalb von 1 Stunde nach dem Aufbringen auf den Arm des Patienten.

Bei dem isolierten 10 : 90-VP/LM-Copolymerisat handelt es sich um ein viskoses, halbfestes Produkt, das in eine Salbe mit einem Gehalt an 10 Prozent VP/LM-Copolymerisat und 4 Prozent Nitroglycerin übergeführt wird. Nach Aufbringen auf den Arm eines Patienten und Abdecken mit einer undurchlässigen Polymerisatfolie, beispielsweise aus Polyäthylen, bewirkt die Salbe eine 24-stündige Beseitigung der Symptome von Angina pectoris.

## Ansprüche

1. Präparat mit verzögerter Freisetzung von Nitroglycerin bei topischer Anwendung auf der Haut, enthaltend ein Gemisch aus Nitroglycerin und einem wasserunlöslichen Vinylpyrrolidon-Polymerisat.

6

2. Präparat nach Anspruch 1, dadurch gekennzeichnet, dass es sich bei dem wasserunlöslichen Vinylpyrrolidon-Polymerisat um ein vernetztes Homopolymerisat handelt.

3. Präparat nach Anspruch 1, dadurch gekennzeichnet, dass es sich bei dem wasserunlöslichen Vinylpyrrolidon-Polymerisat um ein unvernetztes Copolymerisat handelt.

4. Präparat nach Anspruch 3, dadurch gekennzeichnet, dass das unvernetzte Vinylpyrrolidon-Copolymerisat Stuktureinheiten aufweist, die sich durch Copolymerisation eines Esters einer ungesättigten Säure aus der Gruppe Acrylsäure, Methacrylsäure, Crotonsäure, Maleinsäure und entsprechende Anhydride ergeben.

5. Präparat nach Anspruch 1, dadurch gekennzeichnet, dass es sich bei dem wasserunlöslichen Vinylpyrrolidon-Polymerisat um ein vernetztes Copolymerisat handelt.

6. Präparat nach Anspruch 3, dadurch gekennzeichnet, dass das wasserunlösliche Vinylpyrrolidon-Copolymerisat Struktureinheiten aufweist, die sich durch Copolymerisation eines Esters einer gesättigten Säure aus der Gruppe der Vinyl- und Allylester von $C_1$-$C_{40}$-Carbonsäuren ergeben.

7. Präparat nach Anspruch 6, dadurch gekennzeichnet, dass es sich bei dem Ester um Vinylacetat handelt.

8. Präparat nach Anspruch 3, dadurch gekennzeichnet, dass das wasserunlösliche Vinylpyrrolidon-Copolymerisat Styrol enthält.

9. Präparat nach Anspruch 1, dadurch gekennzeichnet, dass das Nitroglycerin und das wasserunlösliche Vinylpyrrolidon-Polymerisat mit einer Salbengrundlage versetzt sind.

10. Präparat nach Anspruch 9, dadurch gekennzeichnet, dass es sich bei der Salbengrundlage um Petrolatum, Lanolin, Stearin und/oder Walrat handelt.

11. Verbandmaterial, Gaze, Binde oder dergleichen, gekennzeichnet durch eine Imprägnierung oder eine Beschichtung mit dem Präparat nach Anspruch 1.

12. Verbandmaterial, Gaze, Binde oder dergleichen, gekennzeichnet durch eine Imprägnierung oder Beschichtung mit dem Präparat nach Anspruch 9.

13. Verfahren zur Herstellung eines Präparats mit verzögerter Freisetzung von Nitroglycerin bei topischer Anwendung auf der Haut, dadurch gekennzeichnet, dass man Nitroglycerin mit einem wasserunlöslichen Vinylpyrrolidon-Homopolymerisat oder -Copolymerisat vermischt.

14. Verfahren zur Herstellung einer Salbe mit verzögerter Freisetzung von Nitroglycerin bei topischer Anwendung auf die Haut, dadurch gekennzeichnet, dass man Nitroglycerin, ein wasserunlösliches Vinylpyrrolidon-Homopolymerisat oder -Copolymerisat und eine Salbengrundlage vermischt.

**Claims**

1. A composition for the gradual release of nitroglycerin upon application to the skin of a patient, comprising a mixture of nitroglycerin and a water-insoluble vinylpyrrolidone polymer.

2. The composition of claim 1, characterized in that the waterinsoluble vinylpyrrolidone polymer is a crosslinked homopolymer.

3. The composition of claim 1, characterized in that the waterinsoluble vinylpyrrolidone polymer is an uncrosslinked copolymer.

4. The composition of claim 3, characterized in that the uncrosslinked vinylpyrrolidone copolymer contains unit derived from the copolymerisation of an ester of an unsaturated acid selected from the group consisting of acrylic acid, methacrylic acid, crotonic acid, maleic acid and the corresponding anhydrides.

5. The composition of claim 1, characterized in that the waterinsoluble vinylpyrrolidone polymer is a crosslinked copolymer.

6. The composition of claim 3, characterized in that the waterinsoluble vinylpyrrolidone copolymer contains units derived from the copolymerization of an ester of a saturated acid selected from the group consisting of the vinyl and allyl esters of $C_1$-$C_{40}$ carboxylic acids.

7. The composition of claim 6, characterized in that the ester is vinyl acetate.

8. The composition of claim 3, characterized in that the water-isoluble vinylpyrrolidone copolymer contains styrene.

9. The composition of claim 1, characterized in that the nitroglycerin and water-insoluble vinylpyrrolidone polymer are combined with an ointment base.

10. The composition of claim 9, characterized in that the ointment base is one or more of the bases selected from the group consisting of petrolatum, lanolin, stearin and spermacetti wax.

11. A bandage, gauze or tape characterized in that it is impregnated or coated with the composition of claim 1.

12. A bandage, gauze or tape characterized in that it is impregnated or coated with the composition of claim 9.

13. A method for preparing a composition capable of gradually releasing nitroglycerin upon topical application to the skin, characterized in that it comprises mixing nitroglycerin with a water-insoluble vinylpyrrolidone homopolymer or copolymer.

14. A method for preparing an ointment capable of gradually releasing nitroglycerin upon topical application to the skin characterized in that it comprises mixing nitroglycerin, a water-insoluble vinylpyrrolidone homopolymer or copolymer and an ointment base.

**Revendications**

1. Composition à libération graduelle de nitroglycérine pour application locale sur la peau, comprenant un mélange de nitroglycérine et d'un polymère de vinylpyrrolidone insoluble dans l'eau.

2. Composition selon la revendication 1, caractérisé par le fait que le polymère de vinylpyrrolidone insoluble dans l'eau est un homopolymère réticulé.

3. Composition selon la revendication 1, caractérisé par le fait que le polymère de vinylpyrrolidone insoluble dans l'eau est un copolymère non réticulé.

4. Composition selon la revendication 3, caractérisé par le fait que le copolymère de vinylpyrrolidone non réticulé contient des motifs dérivés de la copolymérisation d'un ester d'acide insaturé choisi dans le groupe constitué par les acides acryliques, méthacryliques, crotonique, maléique et les anhydrides correspondants.

5. Composition selon la revendication 1, caractérisé par le fait que le polymère de vinylpyrrolidone insoluble dans l'eau est un copolymère réticulé.

6. Composition selon la revendication 3, caractérisé par le fait que le copolymère de vinylpyrrolidone insoluble dans l'eau contient des motifs résultant de la copolymérisation d'un ester d'acide saturé choisi dans le groupe constitué par les esters vinyliques et allyliques d'acides carboxyliques en $C_1$-$C_{40}$.

7. Composition selon revendication 6, caractérisé par le fait que l'ester est l'acétate de vinyle.

8. Composition selon revendication 3, caractérisé par le fait que le copolymère de vinylpyrrolidone insoluble dans l'eau contient du styrène.

9. Composition selon la revendication 1, caractérisé par le fait que la nitroglycérine et le polymère de vinylpyrrolidone insoluble dans l'eau sont combinés avec une base d'onguent.

10. Composition selon la revendication 9, caractérisé par le fait que la base d'onguent est la vaseline, la lanoline, la stéarine et/ou le blanc de baleine.

11. Bandage, gaze, ruban ou similaire caractérisés par le fait qu'ils sont imprégnés ou revêtus de la composition de la revendication 1.

12. Bandage, gaze, ruban ou similaire caractérisés par le fait qu'ils sont imprégnés ou revêtus de la composition de la revendication 9.

13. Procédé de préparation d'une composition à libération graduelle de la nitroglycérine pour application locale sur la peau, caractérisé par le fait qu'on mélange de la nitroglycérine avec un homopolymère ou copolymère de vinylpyrrolidone insoluble dans l'eau.

14. Procédé de préparation d'un onguent à libération graduelle de la nitroglycérine pour application locale sur la peau, caractérisé par le fait qu'on mélange de la nitroglycérine avec un homopolymère ou copolymère de vinylpyrrolidone insoluble dans l'eau et une base d'onguent.